# FASCICULE DE BREVET EUROPEEN

(11) **EP 0 742 097 B1**
(45) Date de publication et mention de la délivrance du brevet: **27.11.2002**
(21) Numéro de dépôt: 96201179.7
(22) Date de dépôt: 29.04.1996
(51) Int. Cl.: B32B 27/08, B32B 1/08, A61L 29/00

(54) **Tube ou feuille multicouche**
Mehrschichtiger Schlauch oder Film
Multilayered film or tube

(30) Priorité: 12.05.1995 BE 9500431
(43) Date de publication de la demande: 13.11.1996
(73) Titulaire: SOLVAY (Société Anonyme), 1050 Bruxelles (BE)
(72) Inventeur: Robben, Petrus A.M, 8225 GC Lelystad (NL); Karsten, Petrus J.A., 1602 NV Enkhuizen (NL); Vonk, Martines W., 1391 GK Abcoude (NL)
(74) Mandataire: Dufrasne, Eugène

(56) Documents cités:
- EP-A- 0 160 984
- EP-A- 0 326 827
- EP-A- 0 380 270
- EP-A- 0 389 695
- EP-A- 0 506 348
- US-A- 3 836 620
- US-A- 5 171 640
- DATABASE WPI Section Ch, Week 8425 Derwent Publications Ltd., London, GB; Class A92, AN 84-156105 XP002009027 & JP-A-59 083 651 ( SUMITOMO BAKELITE KK) , 15 Mai 1984
- PATENT ABSTRACTS OF JAPAN vol. 017, no. 639 (M-1515), 26 Novembre 1993 & JP-A-05 200951 (SHOWA DENKO KK), 10 Août 1993,

## Description

La présente invention concerne un tube ou une feuille multicouche, particulièrement destiné à une utilisation dans le domaine médical.

Des feuilles en matière plastique sont couramment utilisées pour la fabrication de poches destinées en particulier au conditionnement de liquides à usage médical.

Des tubes en matière plastique sont aussi couramment utilisés notamment comme connexions des poches susmentionnées, pour l'introduction ou le prélèvement de matière dans ces poches, mais aussi comme moyens de transport de liquides à usage médical devant par exemple être introduits à l'intérieur du corps humain à des fins thérapeutiques ou d'analyse.

Les matières plastiques utilisables pour ces applications doivent présenter des propriétés spécifiques. Elles doivent notamment être souples et offrir une résistance mécanique suffisante. Elles doivent également être strictement compatibles avec les liquides et/ou avec les matières constitutives des autres éléments (poches, accessoires,...) avec lesquels elles seront en contact. Elles doivent aussi résister aux températures de stérilisation de l'ordre de 120 °C auxquelles sont habituellement traités les articles, et conserver leurs propriétés après un tel traitement.

Des poches à usage médical sont classiquement fabriquées à partir de feuilles à base de polymère de chlorure de vinyle (PVC) souple, c'est-à-dire auquel a généralement été ajouté au moins un plastifiant.

Il est aujourd'hui connu de fabriquer alternativement ces poches à partir de feuilles à base de polyoléfine, sans ajout de plastifiant. On enseigne notamment (EP 216639) d'utiliser pour l'emballage de solutions physiologiques une feuille multicouche comprenant une couche constituée de polyoléfine ou de polyester et une autre couche constituée d'un mélange de polyoléfine et d'un élastomère.

Des tubes en matière plastique adaptée doivent donc être conçus, pour répondre en particulier à l'exigence susmentionnée de compatibilité avec les matières nouvelles constitutives des poches et des accessoires avec lesquels ils doivent être assemblés.

Il est dès lors connu de fabriquer, notamment par coextrusion, des tubes multicouches à usage médical qui peuvent être assemblés avec les poches ou accessoires de manière simple et parfaitement fiable, par exemple par soudage (thermique, haute fréquence,...). Dans le document EP 380270, qui est le document d'art antérieur le plus proche, on décrit un tube tricouche à usage médical, destiné à être assemblé avec une poche ou un autre conditionnement à usage médical, comprenant une couche intermédiaire comprenant un matériau polymérique favorisant la flexibilité, une couche interne liée à la surface interne de la couche intermédiaire et comprenant un matériau résistant à la chaleur, et une couche externe liée à la surface externe de la couche intermédiaire et comprenant du polypropylène, un copolymère d'éthylène et de propylène ou un copolymère modifié d'éthylène et de propylène. Selon ce document, le matériau polymérique de la couche intermédiaire peut en particulier comprendre du polyéthylène de très faible densité, un copolymère d'éthylène et d'acétate de vinyle; un copolymère modifié d'éthylène et d'acétate de vinyle, un copolymère d'éthylène et d'acrylate de méthyle, un copolymère modifié d'éthylène et d'acrylate de méthyle, un polymère du chlorure de vinyle, un polymère du chlorure de vinylidène, un mélange d'un copolymère d'éthylène et d'acétate de vinyle et d'un copolymère modifié d'éthylène et d'acrylate de méthyle, un mélange de polyéthylène de très faible densité et d'un copolymère modifié d'éthylène et d'acrylate de méthyle, ou un mélange de polyéthylène de très faible densité et d'un copolymère d'éthylène et d'acétate de vinyle.

Outre les exigences susmentionnées, les tubes multicouches à usage médical doivent présenter en particulier une résistance suffisante à la délamination de leurs couches constitutives, notamment après le traitement thermique de stérilisation et après leur assemblage avec d'autres éléments.

La présente invention a dès lors pour premier objet un tube multicouche destiné notamment à un usage médical, satisfaisant aux exigences susmentionnées et offrant en particulier une résistance très élevée à la délamination de ses couches constitutives.

L'invention concerne plus précisément un tube multicouche comprenant une couche intermédiaire, une couche interne liée à la surface interne de la couche intermédiaire et une couche externe liée à la surface externe de la couche intermédiaire, qui se caractérise en ce que la couche intermédiaire comprend essentiellement un matériau polymérique choisi parmi les terpolymères d'éthylène, d'anhydride maléique et d'un acrylate de formule H₂C=CHCO₂R, dans laquelle R est de formule CₓH₍₂ₓ₊₁₎ avec x=2 et en ce que la couche interne ou la couche externe comprend un matériau polymérique choisi parmi (I) les polymères plastifiés du chlorure de vinyle, les polyuréthanes, les copolymères à blocs de polyéther et d'amide ou les mélanges d'un copolymère d'éthylène et d'acétate de vinyle avec un copolyester, l'autre couche interne ou externe comprenant un matériau polymérique choisi parmi (II) le polypropylène, les copolymères d'éthylène et de propylène, les copolymères modifiés d'éthylène et de propylène, les mélanges ternaires de polypropylène ou d'un copolymère d'éthylène et de propylène ou d'un copolymère modifié d'éthylène et de propylène avec un copolymère d'éthylène et d'un ester acrylique et avec un copolymère à blocs de styrène-éthylène-butylène-styrène.

Le tube multicouche comprend une couche intermédiaire et au moins deux autres couches, une couche interne et une couche externe. Il peut éventuellement comprendre une ou plusieurs autres couches supplémentaires. De préférence, la couche intermédiaire est directement liée à la couche interne et à la couche externe. D'excellents résultats ont été obtenus avec un tube constitué des trois couches susmentionnées.

La couche intermédiaire comprend essentiellement un matériau polymérique choisi parmi les terpolymères d'éthylène, d'anhydride maléique et d'un acrylate de formule H₂C=CHCO₂R, dans laquelle R est de formule CₓH₍₂ₓ₊₁₎ avec x=2.

L'épaisseur relative de la couche intermédiaire doit être adaptée aux matériaux mis en oeuvre dans les différentes couches et aux contraintes ultérieures d'utilisation du tube multicouche. La couche intermédiaire a généralement une épaisseur correspondant au moins à 1 % de l'épaisseur totale de la paroi du tube. De préférence, son épaisseur correspond au moins à 5 % de l'épaisseur totale de la paroi du tube. Plus préférentiellement encore, son épaisseur correspond au moins à 15 % de l'épaisseur totale de la paroi du tube. L'épaisseur de la couche intermédiaire ne dépasse le plus souvent pas 99 % de l'épaisseur totale de la paroi du tube. De manière avantageuse, cette épaisseur correspond au plus à 50 % de l'épaisseur totale de la paroi du tube. Plus avantageusement encore, cette épaisseur correspond au plus à 30 % de l'épaisseur totale de la paroi du tube.

Les matériaux mis en oeuvre dans la couche interne et/ou dans la couche externe doivent également être adaptés aux matériaux de la ou des autres couches, aux contraintes de mise en oeuvre et aux contraintes ultérieures d'utilisation du tube multicouche.

Selon un premier mode d'utilisation du tube multicouche dans le domaine médical, il peut être assemblé par sa surface extérieure avec une poche à usage médical afin de recevoir en son intérieur un accessoire devant être connecté avec la poche.

Selon un second mode d'utilisation, le tube multicouche peut être assemblé par sa surface extérieure avec un accessoire et transporter en son intérieur un liquide à usage médical.

Il est nécessaire que la ou les couches devant être en contact avec un accessoire comprennent un matériau polymérique compatible avec cet accessoire. A cette fin, chaque couche concernée, soit au moins la couche interne ou la couche externe comprend un matériau polymérique choisi parmi (I) les polymères plastifiés du chlorure de vinyle, les polyuréthanes, les copolymères à blocs de polyéther et d'amide ou les mélanges d'un copolymère d'éthylène et d'acétate de vinyle avec un copolyester. De manière particulièrement préférée, chaque couche concernée est essentiellement constituée d'un tel matériau polymérique.

On recommande aussi que la ou les couches devant être en contact avec une poche ou avec un liquide à usage médical comprennent un matériau polymérique compatible avec l'élément correspondant. A cette fin, chaque couche concernée, soit au moins la couche interne ou la couche externe comprend un matériau polymérique choisi parmi (II) le polypropylène, les copolymères d'éthylène et de propylène, les copolymères modifiés d'éthylène et de propylène, les mélanges ternaires de polypropylène ou d'un copolymère d'éthylène et de propylène ou d'un copolymère modifié d'éthylène et de propylène avec un copolymère d'éthylène et d'un ester acrylique et avec un copolymère à blocs de styrène-éthylène-butylène-styrène. De manière particulièrement avantageuse, chaque couche concernée est essentiellement constituée d'un tel matériau polymérique.

En particulier pour un tube multicouche destiné au premier mode d'utilisation tel que défini ci-dessus, il est intéressant de retenir la couche interne comprenant un matériau polymérique choisi parmi les polymères (I) et la couche externe comprenant un matériau polymérique choisi parmi les polymères (II). De manière particulièrement intéressante, la couche interne et la couche externe sont essentiellement constituées de ces matériaux polymériques .

Dans le cas notamment d'un tube destiné au second mode d'utilisation tel que défini ci-dessus, il est à l'inverse intéressant de retenir la couche interne comprenant un matériau polymérique choisi parmi les polymères (II) et la couche externe comprenant un matériau polymèrique choisi parmi les polymères (I). De manière particulièrement intéressante, la couche interne et la couche externe sont essentiellement constituées de ces matériaux polymériques .

Le tube multicouche selon l'invention est particulièrement adapté à une utilisation dans le domaine médical, notamment en ce qu'il satisfait aux exigences susmentionnées à cet effet et en ce qu'il offre une résistance très élevée à la délamination de ses couches constitutives, notamment après le traitement thermique de stérilisation et après son assemblage éventuel avec d'autres éléments.

La présente invention concerne dès lors également l'utilisation dans le domaine médical du tube multicouche tel que défini ci-dessus.

Vu les propriétés particulièrement intéressantes de la structure multicouche du tube défini ci-avant, cette structure peut aussi être avantageusement retenue pour une feuille multicouche.

L'invention a dès lors également pour objet une feuille multicouche comprenant des couches telles que définies ci-avant dans le tube multicouche.

L'invention concerne enfin l'utilisation dans le domaine médical de cette feuille multicouche.

On notera enfin que les propriétés des tubes et des feuilles multicouches selon l'invention peuvent éventuellement être améliorées, en particulier en vue de leur stérilisation, en soumettant ces produits à un traitement d'irradiation par un faisceau d'électrons et/ou d'irradiation électromagnétique et/ou par l'incorporation d'un ou plusieurs agents de réticulation usuels tels que par exemple des peroxydés.

### Exemples

On a fabriqué par coextrusion des tubes d'un diamètre extérieur de 8 mm constitués de trois couches de compositions telles que reprises dans les tableaux ci-après.

Les exemples 1R à 4R sont donnés à titre de comparaison.

Les exemples 5 à 9 illustrent l'invention de manière non limitative.

On a ensuite mesuré la résistance à la délamination de chaque tube, avant et après un traitement de stérilisation à la vapeur à 121 °C durant 30 minutes.

Pour mesurer cette résistance à la délamination, on a pris pour chaque tube un tronçon d'une longueur d'approximativement 10 cm. On a coupé chaque tronçon de tube dans le sens de la longueur en 2 ou 3 pièces. Manuellement, on a séparé les deux couches ayant la plus faible adhésion (selon tous ces exemples, la couche en PVC plastifié et la couche intermédiaire) sur une longueur d'environ 2 cm à partir d'une extrémité de chaque pièce. On a ensuite placé les couches partiellement séparées dans les mâchoires d'un appareil de mesure de tension, et on a délaminé chaque pièce à une vitesse constante d'écartement des deux parties de la mâchoire de 100 mm/min, en mesurant la force requise à cet effet. On a alors relevé la valeur représentative de cette force [N] lorsqu'elle était devenue constante, que l'on a divisée par la largeur [mm] de chaque pièce testée pour obtenir la résistance [N/mm] à la délamination mentionnée dans les tableaux ci-après.

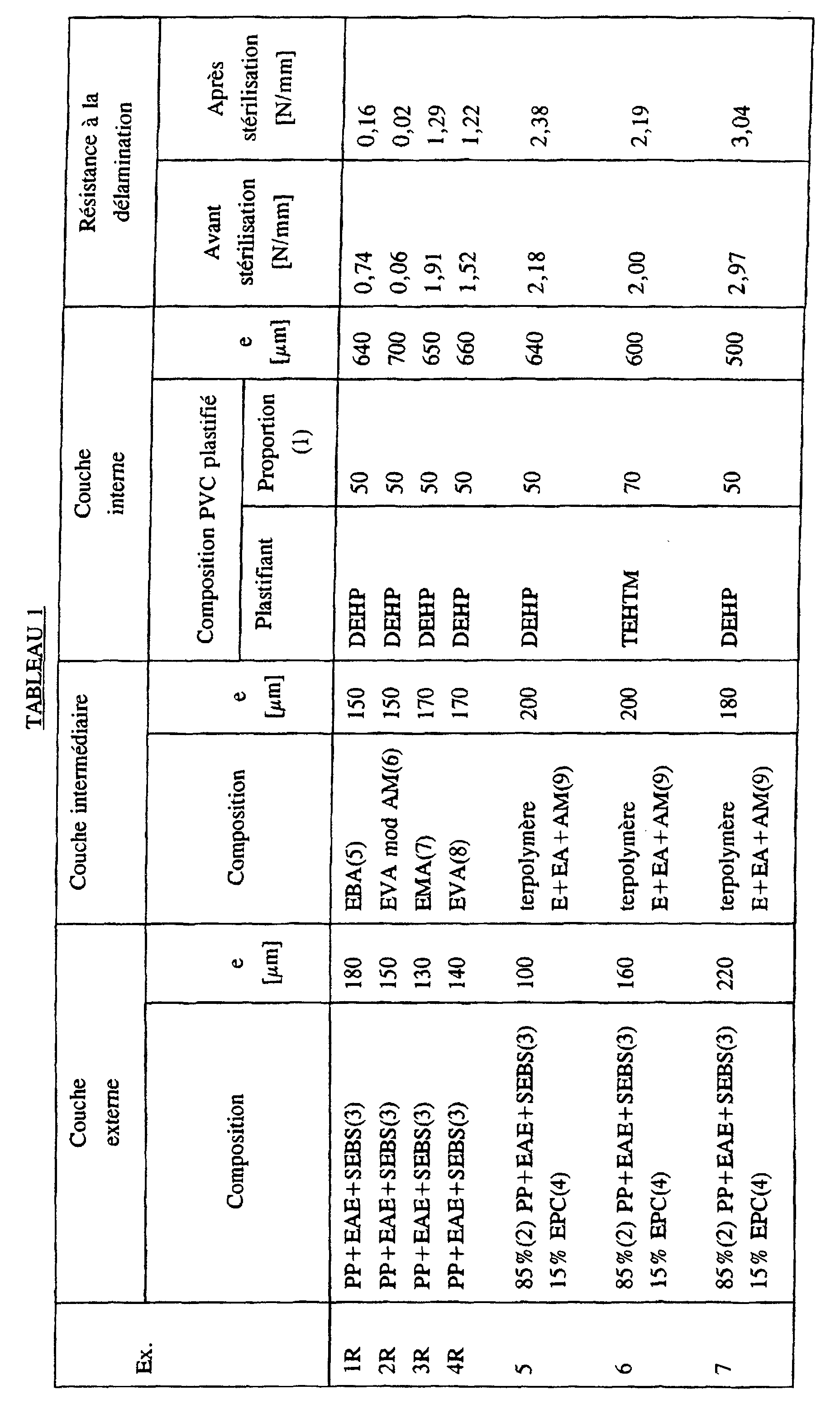

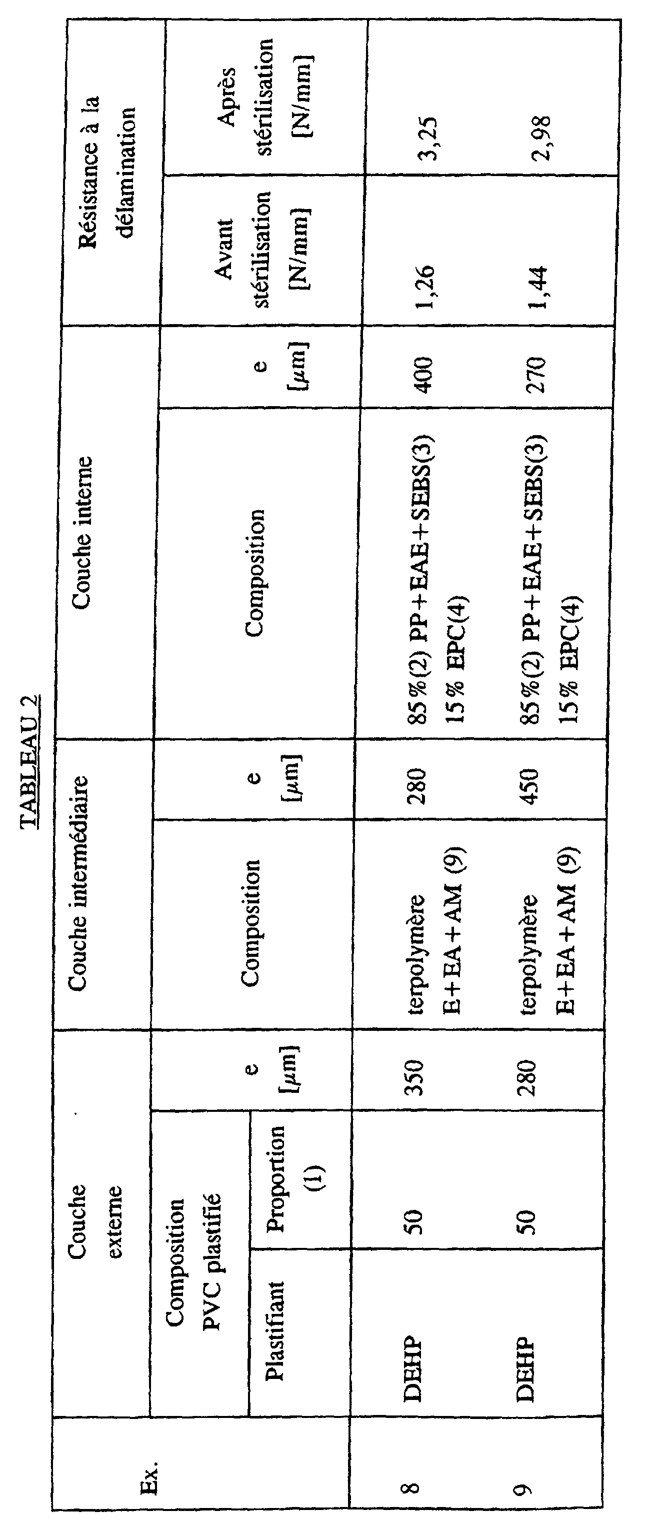

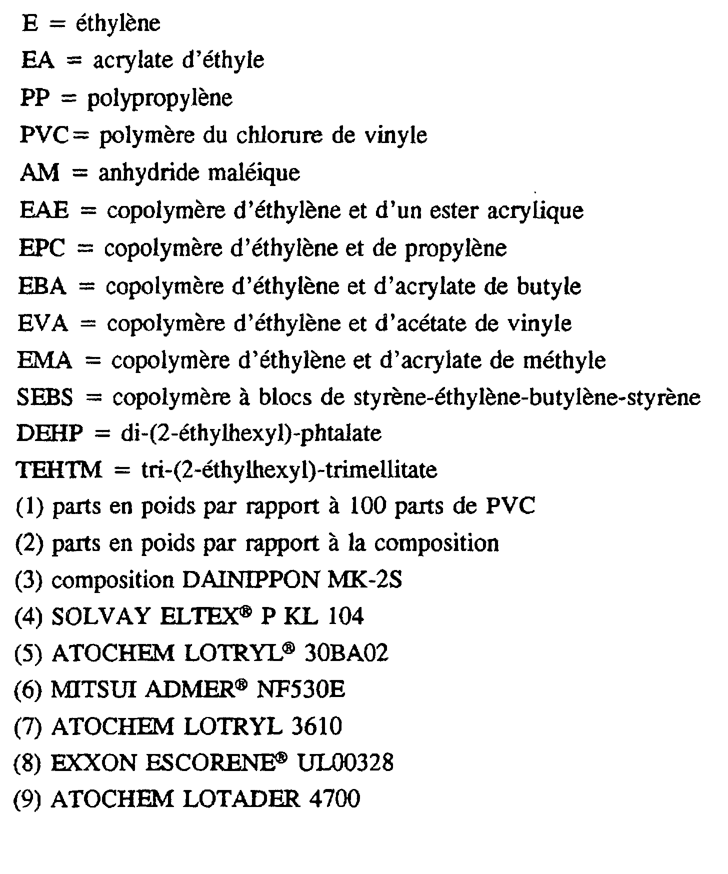

## Revendications

1. Tube multicouche comprenant une couche intermédiaire, une couche interne liée à la surface interne de la couche intermédiaire et une couche externe liée à la surface externe de la couche intermédiaire, **caractérisé en ce que** la couche intermédiaire comprend essentiellement un matériau polymérique choisi parmi les terpolymères d'éthylène, d'anhydride maléique et d'un acrylate de formule H₂C=CHCO₂R, dans laquelle R est de formule CₓH₍₂ₓ₊₁₎ avec x=2 et **en ce que** la couche interne ou la couche externe comprend un matériau polymérique choisi parmi (I) les polymères plastifiés du chlorure de vinyle, les polyuréthanes, les copolymères à blocs de polyéther et d'amide ou les mélanges d'un copolymère d'éthylène et d'acétate de vinyle avec un copolyester, l'autre couche interne ou externe comprenant un matériau polymérique choisi parmi (II) le polypropylène, les copolymères d'éthylène et de propylène, les copolymères modifiés d'éthylène et de propylène, les mélanges ternaires de polypropylène ou d'un copolymère d'éthylène et de propylène ou d'un copolymère modifié d'éthylène et de propylène avec un copolymère d'éthylène et d'un ester acrylique et avec un copolymère à blocs de styrène-éthylène-butylène-styrène.

2. Tube multicouche selon la revendication 1, dans lequel la couche interne comprend un matériau polymérique choisi parmi les polymères (I) et la couche externe comprend un matériau polymérique choisi parmi les polymères (II).

3. Tube multicouche selon la revendication 1, dans lequel la couche interne comprend un matériau polymérique choisi parmi les polymères (II) et la couche externe comprend un matériau polymérique choisi parmi les polymères (I).

4. Utilisation dans le domaine médical du tube multicouche selon l'une quelconque des revendications précédentes.

5. Feuille multicouche comprenant des couches telles que définies dans le tube multicouche selon l'une quelconque des revendications 1 à 3.

6. Utilisation dans le domaine médical de la feuille multicouche selon la revendication 5.

7. Tube ou feuille multicouche selon l'une quelconque des revendications 1 à 3 ou 5, **caractérisé en outre en ce qu'**il a été soumis à un traitement d'irradiation par un faisceau d'électrons et/ou d'irradiation électromagnétique et/ou **en ce qu'**il comprend un ou plusieurs agents de réticulation usuels.

## Claims

1. Multilayer tube comprising an interlayer, an inner layer bonded to the inner surface of the interlayer, and an outer layer bonded to the outer surface of the interlayer, **characterized in that** the interlayer essentially comprises a polymeric material chosen from terpolymers of ethylene, maleic anhydride and an acrylate of formula H₂C=CHCO₂R, in which R is of formula CₓH₍₂ₓ₊₁₎ with x = 2, and **in that** the inner layer or the outer layer comprises a polymeric material chosen from (I) plasticized vinyl chloride polymers, polyurethanes, copolymers having polyether and amide blocks or blends of an ethylene/vinyl acetate copolymer with a copolyester, the other, inner or outer layer comprising a polymeric material chosen from (II) polypropylene, ethylene-propylene copolymers, modified ethylene-propylene copolymers, ternary blends of polypropylene or an ethylene-propylene copolymer or a modified ethylene-propylene copolymer with an ethylene/acrylic ester copolymer and with a styrene/ethylene-butylene/styrene block copolymer.

2. Multilayer tube according to Claim 1, in which the inner layer comprises a polymeric material chosen from the polymers (I) and the outer layer comprises a polymeric material chosen from the polymers (II).

3. Multilayer tube according to Claim 1, in which the inner layer comprises a polymeric material chosen from the polymers (II) and the outer layer comprises a polymeric material chosen from the polymers (I).

4. Use of the multilayer tube according to any one of the preceding claims in the medical field.

5. Multilayer sheet comprising layers as defined in the multilayer tube according to any one of Claims 1 to 3.

6. Use of the multilayer sheet according to Claim 5 in the medical field.

7. Multilayer tube or sheet according to any one of Claims 1 to 3 or 5, **characterized in that** it has been subjected to an irradiation treatment by an electron beam and/or a beam of electromagnetic irradiation and/or **in that** it includes one or more standard crosslinking agents.

## Patentansprüche

1. Mehrschichtiger Schlauch umfassend eine Zwischenschicht, eine Innenschicht, die mit der inneren Fläche der Zwischenschicht verbunden ist, und eine Außenschicht, die mit der äußeren Fläche der Zwischenschicht verbunden ist, **dadurch gekennzeichnet, dass** die Zwischenschicht im Wesentlichen ein Polymer umfasst, das ausgewählt ist unter den Terpolymeren von Ethylen, Maleinsäureanhydrid und eines Acrylats der Formel H₂C=CHCO₂R, worin R die Formel CₓH₍₂ₓ₊₁₎ mit x = 2 besitzt, und dadurch, dass die Innenschicht oder die Außenschicht ein Polymer umfasst, das ausgewählt ist unter (I) den weichgemachten Vinylchloridpolymeren, den Polyurethanen, den Blockcopolymeren eines Polyethers und eines Amids oder den Gemischen eines Ethylen- und Vinylacetatcopolymers mit einem Copolyester, wobei die andere Innen- oder Außenschicht ein Polymer umfasst, das ausgewählt ist unter (II) Polypropylen, den Ethylen- und Propylencopolymeren, den modifizierten Ethylen- und Propylencopolymeren, den ternären Gemischen von Polypropylen oder eines Ethylen- und Propylencopolymers oder eines modifizierten Ethylen- und Propylencopolymers mit einem Copolymer von Ethylen und einem Acrylester und mit einem Styren-Ethylen-Butylen-Styren-Blockcopolymer.

2. Mehrschichtiger Schlauch gemäß Anspruch 1, worin die Innenschicht ein Polymer umfasst, das ausgewählt ist unter den Polymeren (I), und die Außenschicht ein Polymer umfasst, das ausgewählt ist unter den Polymeren (II).

3. Mehrschichtiger Schlauch gemäß Anspruch 1, worin die Innenschicht ein Polymer umfasst, das ausgewählt ist unter den Polymeren (II), und die Außenschicht ein Polymer umfasst, das ausgewählt ist unter den Polymeren (I).

4. Verwendung im medizinischen Bereich des mehrschichtigen Schlauchs gemäß einem den vorhergehenden Ansprüche.

5. Mehrschichtiger Film umfassend Schichten, wie in dem mehrschichtigen Schlauch gemäß einem der Ansprüche 1 bis 3 definiert.

6. Verwendung im medizinischen Bereich des mehrschichtigen Films gemäß Anspruch 5.

7. Mehrschichtiger Schlauch oder Film gemäß einem der Ansprüche 1 bis 3 oder 5, außerdem **dadurch gekennzeichnet, dass** er einer Behandlung der Bestrahlung mit einem Elektronenstrahl und/oder der elektromagnetischen Bestrahlung unterzogen wurde, und/oder dadurch, dass er ein oder mehrere übliche Vernetzungsmittel umfasst.
